# NOUVEAU FASCICULE DE BREVET EUROPEEN

(11) **EP 1 302 190 B2**
(45) Date de publication et mention de la décision concernant l'opposition: **07.04.2010**
(45) Mention de la délivrance du brevet: 20.12.2006
(21) Numéro de dépôt: 02292332.0
(22) Date de dépôt: 23.09.2002
(51) Int. Cl.: A61K 8/06, A61K 8/37, A61K 8/89, A61K 8/31, A61K 8/81, A61Q 1/14

(54) **Composition cosmétique de démaquillage et/ou de nettoyage de la peau**
Kosmetische Zusammensetzung für das Abschminken oder die Hautreinigung
Cosmetic composition for make-up removal or skin cleansing

(30) Priorité: 15.10.2001 FR 0113271; 22.08.2002 FR 0210494
(43) Date de publication de la demande: 16.04.2003
(73) Titulaire: L'Oréal, 75008 Paris (FR)
(72) Inventeur: Lennon, Paula, 69003 Lyon (FR); Boschet, Cécile, 94550 Chevilly-Larue (FR); Guiramand, Carole, 78350 Jouy-en-Josas (FR)
(74) Mandataire: Rasson, Catherine

(56) Documents cités:
- EP-A- 0 510 580
- EP-A- 1 055 406
- EP-A1- 0 494 022
- EP-A1- 0 835 647
- EP-A2- 0 503 853
- EP-A2- 0 750 899
- EP-A2- 1 236 464
- WO-A1-01/35922
- WO-A1-92/06778
- WO-A2-02/43686
- WO-A2-02/44270
- DE-A- 10 010 851
- US- - 5 470 551
- US-A- 5 612 429
- DATABASE CHEMICAL ABSTRACTS [en ligne] retrieved from STN Database accession no. 127:298557 XP002205670 & JP 09 175938 A (KOSEI K.K.) 8 juillet 1997 (1997-07-08)
- JOHN A. WENNINGER AND G.N. MCEWEN(EDITORS): "International Cosmetic Ingredient Dictionary and Handbook" VOLUME 2, THE COSMETIC, TOILETRY, AND FRAGRANCE ASSOCIATION , WASHINGTON DC, US Entry for PEG-60 HYDROGENATED CASTOR OIL
- JOHN A. WENNINGER, RENAE C.CANTERBERY,G.N.MCEWEN, JR.,PH.D.,J.D.: 'International Cosmetic Ingredient Dictionary and Handbook', vol. 1, 2000, COSMETIC, TOILETRY, AND FRAGANCE ASSOCIATION article EIGHTH EDITION, pages 24 - 24

## Description

L'invention se rapporte à une composition cosmétique de démaquillage et/ou de nettoyage se présentant sous forme d'une émulsion huile-dans-eau comportant un polymère amphiphile particulier et au moins une huile démaquillante. Cette composition est utilisée notamment pour le démaquillage et/ou le nettoyage de la peau, des lèvres et/ou des yeux.

Les démaquillants et nettoyants du visage se présentant sous forme d'émulsions, classiquement utilisés à ce jour présentent des inconvénients qui ne sont pas les mêmes selon le sens de l'émulsion. Dans le cas des émulsions eau-dans-huile, très efficaces pour le démaquillage/nettoyage du fait que la phase grasse est directement disponible pour dissoudre les différents corps gras présents sur la peau ou issus du leur utilisation est inconfortable du fait de la sensation grasse et lourde apportée par cette phase grasse externe qui subsiste sur la peau.

Dans le cas des émulsions huile-dans-eau (H/E), la phase aqueuse externe apporte de la fraîcheur. En revanche, la phase huileuse étant la phase interne, elle n'est pas directement disponible pour dissoudre les corps gras et, de ce fait, le démaquillage est moins efficace. Or, pour le nettoyage du visage et plus spécialement pour le démaquillage qui consiste à enlever tous les produits de maquillage, les femmes recherchent à obtenir une démaquillage le plus efficace possible.

Pour améliorer l'efficacité d'une composition démaquillante sous forme d'émulsion H/E, il est nécessaire d'introduire une quantité importante de phase grasse (phase huileuse), puisque c'est cette dernière qui assure un bonne dissolution des particules solides qui composent le maquillage. Toutefois, quand on introduit une quantité importante d'huiles dans une phase aqueuse gélifiée par un gélifiant aqueux classique, la stabilité de la composition se trouve compromise au delà d'une certaine quantité d'huile, et il est alors nécessaire d'ajouter un tensioactif émulsionnant, qui présent en une quantité suffisante, va stabiliser l'émulsion. Toutefois, pour des raisons d'innocuité, en particulier quand la composition est utilisée pour le démaquillage des yeux, il est important d'utiliser des émulsions qui ne comportent pas de tensioactifs émulsionnants. Pour assurer la stabilité des émulsions sans tensioactif émulsionnant, il est connu de gélifier la phase aqueuse externe. Toutefois, quand on introduit une quantité importante d'huiles dans une phase aqueuse gélifiée par un gélifiant aqueux classique, la stabilité de la composition se trouve compromise au delà d'une certaine quantité d'huile si l'on n'ajoute pas en plus un tensioactif émulsionnant. Et il est d'autant plus difficile de réaliser des émulsions sans tensioactif émulsionnant avec un fort taux d'huile, que le milieu est fluide.

Par ailleurs, si certains gélifiants tels que les copolymères acryliques réticulés (Pemulen par exemple) permettent d'émulsionner plus de 20 % en poids d'huile, les compositions obtenues ont de médiocres propriétés cosmétiques car le toucher en est lourd et collant, donc peu apprécié des utilisateurs.

EP-A-1 055 406 concerne une composition cosmétique sous forme d'émulsion huile-dans-eau qui contient dans un milieu physiologiquement acceptable (i) au moins un organopolysiloxane solide élastomère réticulé comportant au moins un groupement oxyalkyléné et (ii) au moins un polymère hydrosoluble ou gonflable pour remplacer le tensioactif émulsionnant normalement employé.

Aussi, il subsiste le besoin d'une composition cosmétique nettoyante et/ou démaquillante sous forme d'émulsion huile-dans-eau, qui soit stable et efficace tout en ne contenant pas de tensioactif émulsionnant et en ayant de bonnes propriétés cosmétiques.

La demanderesse a maintenant trouvé un polymère amphiphile permettant d'atteindre ces objectifs, et ce quelle que soit le viscosité de la composition, c'est-à-dire qu'elle soit fluide ou épaisse.

La présente invention a pour objet une composition cosmétique de nettoyage et/ou de démaquillage, comprenant une phase huileuse dispersée dans une phase aqueuse, caractérisée en ce qu'elle comprend au moins un polymère amphiphile comportant au moins un monomère à insaturation éthylénique à groupement sulfonique, sous forme libre ou partiellement ou totalement neutralisée, non réticulé, comportant au moins deux parties latérales hydrophobes, et que la phase huileuse comprend au moins une huile démaquillante et constitue au moins 40 % en poids par rapport au poids total de la composition, le polymère amphiphile étant choisi parmi les copolymères amphiphiles d'AMPS d'au moins un monomère hydrophobe à insaturation éthylènique comportant au moins une partie hydrophobe ayant de 6 à 50 atomes de carbone et de préférence de 6 à 30 atomes de carbone.

La composition étant une composition cosmétique, elle comprend un milieu physiologiquement acceptable. On entend dans la présente demande par « milieu physiologiquement acceptable », un milieu compatible avec toutes les matières kératiniques telles que la peau y compris le cuir chevelu, les ongles, les muqueuses, les yeux et les cheveux ou toute autre zone cutanée du corps.

La composition de l'invention, même exempte de tout agent émulsionnant, présente une excellente stabilité, quelle que soit sa viscosité et quelle que soit la teneur en huiles, et, en outre, elle présente une très bonne efficacité de démaquillage. Par ailleurs, le toucher cosmétique de la composition selon l'invention est très agréable : il n'y a aucun effet collant, et elle est fraîche lors de l'application sur la peau.

La composition selon l'invention présente de préférence une viscosité allant de 0,1 Pa.s (soit 1 poises) à 23 Pa.s (230 poises), de préférence de 0,1 Pa.s (1 poises) à 15 Pa.s. (150 poises), et mieux de 0,2 à 15 Pa.s (2 à 150 poises), cette viscosité étant mesurée à la température ambiante (25°C) avec l'appareil RM180 Rheomat de la société METTLER.

La composition selon l'invention a généralement un pH allant de 3 à 9, mieux de 4 à 9, et mieux de 4,5 à 8 et encore mieux de 6 à 8.

### Polymères amphiphiles

Les polymères utilisés conformément à l'invention sont des polymères amphiphiles non réticulés comportant au moins un monomère à insaturation éthylénique à groupement sulfonique, sous forme libre ou partiellement ou totalement neutralisée et comprenant au moins deux parties latérales hydrophobes.

On entend par polymère amphiphile, tout polymère comportant à la fois une partie hydrophile et une partie hydrophobe et notamment une chaîne grasse.

Le polymère utilisé dans la composition de l'invention est un polymère hydrophile. On entend par polymère hydrophile, tout polymère soluble ou dispersible dans l'eau, tel quel ou sous forme neutralisée.

La partie hydrophobe présente dans les polymères de l'invention comporte de 6 à 50 atomes de carbone, plus préférentiellement de 6 à 30, encore mieux de 6 à 22 atomes de carbone, plus préférentiellement encore de 6 à 18 atomes de carbone, et plus particulièrement de 12 à 18 atomes de carbone.

Les polymères utilisés conformément à l'invention sont des polymères amphiphiles susceptibles d'être obtenus à partir d'au moins un monomère à insaturation éthylénique à groupement sulfonique, sous forme libre ou partiellement ou totalement neutralisée, et d'au moins un monomère hydrophobe.

De façon préférentielle, les polymères conformes à l'invention sont neutralisés partiellement ou totalement par une base cosmétiquement acceptable qui peut être une base minérale (soude, potasse, ammoniaque) ou une base organique telle que la mono-, di- ou tri-éthanolamine, un aminométhylpropanediol, la N-méthyl-glucamine, les acides aminés basiques comme l'arginine et la lysine, et les mélanges de ces composés.

Les polymères amphiphiles conformes à l'invention ont généralement un poids moléculaire moyen en nombre allant de 1000 à 20 000 000 g/mole, de préférence allant de 20 000 à 5 000 000 et plus préférentiellement encore de 100 000 à 1 500 000 g/mole.

Les polymères amphiphiles selon l'invention sont non-réticulés.

On entend par polymère non réticulé, un polymère n'ayant pas réagi avec des agents de réticulation et donc ne comportant pas de groupes de réticulation. Il peut s'agir d'un polymère linéaire ou branché. Les polymères non réticulés utilisés dans la composition de l'invention sont de préférence linéaires.

Les polymères non réticulés de l'invention sont choisis parmi les copolymères amphiphiles d'AMPS et d'au moins un monomère hydrophobe à insaturation éthylénique comportant au moins une partie hydrophobe ayant de 6 à 50 atomes de carbone, plus préférentiellement de 6 à 22 atomes de carbone, encore plus préférentiellement de 6 à 18 atomes de carbone et plus particulièrement 12 à 18 atomes de carbone. A côté de ces monomères, ces copolymères peuvent contenir en outre un ou plusieurs monomères éthyléniquement insaturés ne comportant pas de chaîne grasse (hydrophobe), tels que les acides (méth)acryliques, leurs dérivés alkyl substitués en β ou leurs esters obtenus avec des monoalcools ou des mono- ou polyalkylèneglycols, les (méth)acrylamides, la vinylpyrrolidone, l'anhydride maléique, l'acide itaconique, l'acide maléique, des monomères cationiques tels que le chlorure de diallyl diméthylammonium, ou les mélanges de ces composés.

Ces copolymères sont décrits notamment dans les documents EP-A-750899, US-A-5089578 et dans les publications de Yotaro Morishima suivantes :
- « Self-assembling amphiphilic polyelectrolytes and their nanostructures - Chinese Journal of Polymer Science Vol. 18, N°40, (2000), 323-336 »;
- « Miscelle formation of random copolymers of sodium 2-(acrylamido)-2-methylpropanesulfonate and a non-ionic surfactant macromonomer in water as studied by fluorescence and dynamic light scattering - Macromolecules 2000, Vol. 33, N° 10 - 3694-3704 » ;
- « Solution properties of miscelle networks formed by non-ionic moieties covalently bound to an polyelectrolyte : salt effects on rheological behavior - Langmuir, 2000, Vol.16, N°12, 5324-5332 » ;
- « Stimuli responsive amphiphilic copolymers of sodium 2-(acrylamido)-2-methylpropanesulfonate and associative macromonomers - Polym. Preprint, Div. Polym. Chem. 1999, 40(2), 220-221 ».

Les monomères hydrophobes à insaturation éthylénique de ces copolymères particuliers sont choisis de préférence parmi les acrylates, alkylacrylates, acrylamides ou alkylacrylamides de formule (I) suivante : 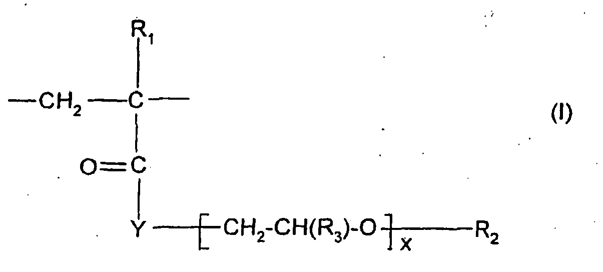 dans laquelle R₁ et R₃, identiques ou différents, désignent un atome d'hydrogène
ou un radical alkyle linéaire ou ramifié en C₁-C₆ (de préférence méthyle) ; Y désigne O ou NH ; R₂ désigne un radical hydrocarboné hydrophobe comportant au moins 6 atomes de carbone, de préférence de 6 à 50 atomes de carbone, plus préférentiellement de 6 à 30 atomes de carbone, plus préférentiellement encore de 6 à 22 atomes de carbone, encore plus préférentiellement de 6 à 18 atomes de carbone et plus particulièrement de 12 à 18 atomes de carbone ; x désigne un nombre de moles d'oxyde d'alkylène et varie de 0 à 100.

Le radical R₂ est choisi de préférence parmi les radicaux alkyles en C₆-C₁₈ linéaires (par exemple n-hexyle, n-octyle, n-décyle, n-hexadécyle, n-dodécyle, n-octadécyle, n-béhényle, oléyle), ramifiés (par exemple isostéaryle) ou cycliques (par exemple cyclododécyle (C₁₂) ou adamantyle (C₁₀)) ; les radicaux alkylperfluorés en C₆-C₃₀ et de préférence en C₆-C₁₈ (par exemple le groupement de formule -(CH₂)₂-(CF₂)₉-CF₃); le radical cholestéryle (C₂₇) ou un reste d'ester de cholestérol comme le groupe oxyhexanoate de cholestéryle ; les groupes polycycliques aromatiques comme le naphtalène ou le pyrène, les radicaux siliconés ou alkylsiliconés ou encore alkylfluorosiliconés. Parmi ces radicaux, on préfère plus particulièrement les radicaux alkyles linéaires et plus particulièrement le radical n-dodécyle, n-hexadecyle, n-octadécyle et leurs mélanges.

Selon une forme particulièrement préférée de l'invention, le monomère de formule (I) comporte au moins un motif oxyde d'alkylène (x ≥ 1) et de préférence plusieurs motifs oxyde d'alkylène (x>1) constituant une chaîne polyoxyalkylénée. La chaîne polyoxyalkylénée, de façon préférentielle, est constituée de motifs oxyde d'éthylène et/ou de motifs oxyde de propylène et encore plus particulièrement constituée de motifs oxyde d'éthylène. Le nombre de motifs oxyalkylénés (ou nombre de motifs oxyde d'alkylène) varie en général de 3 à 100, plus préférentiellement de 3 à 50 et encore plus préférentiellement de 7 à 25.

Parmi ces polymères, on peut citer :
- les copolymères non réticulés, neutralisés ou non, comportant de 15 à 60 % en poids de motifs AMPS et de 40 à 85 % en poids de motifs (C₈-C₁₆)alkyl(méth)acrylamide ou de motifs (C₈-C₁₆)alkyl(méth)acrylate par rapport au polymère, tels que ceux décrits dans la demande EP-A-750 899 ;
- les terpolymères comportant de 10 à 90% en mole de motifs acrylamide, de 0,1 à 10% en mole de motifs AMPS et de 5 à 80% en mole de motifs N-(C₆-C₁₈)alkylacrylamide, tels que ceux décrits dans le brevet US-A-5,089,578.

On peut également citer les copolymères non réticulés, d'AMPS partiellement ou totalement neutralisé et de méthacrylate de dodécyle, de n-hexadécyle et/ou de n-octadécyle, ainsi que les copolymères non-réticulés d'AMPS et de n-dodécylméthacrylamide, tels que ceux décrits dans les articles de Morishima cités ci-dessus.

On peut citer plus particulièrement les copolymères non réticulés constitués de motifs acide 2-acrylamido-2-méthylpropane-sulfonique (AMPS) de formule (II) suivante : 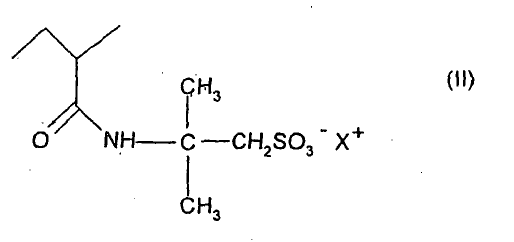 dans laquelle X⁺ est un proton, un cation de métal alcalin, un cation alcalino-terreux ou l'ion ammonium,
et de motifs de formule (III) suivante : 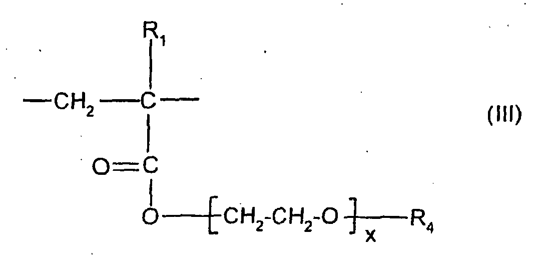 dans laquelle x désigne un nombre entier variant de 3 à 100, de préférence de 5 à 80 et plus préférentiellement de 7 à 25 ; R₁ a la même signification que celle indiquée ci-dessus dans la formule (I) et R₄ désigne un alkyle linéaire ou ramifié en C₆-C₂₂ et plus préférentiellement en C₁₀-C₂₂.

Les polymères particulièrement préférés sont ceux pour lesquels x = 25, R₁ désigne méthyle (CH₃) et R₄ représente n-dodécyle ; ils sont décrits dans les articles de Morishima mentionnés ci-dessus. D'autres polymères préférés sont ceux pour lesquels x = 25, R₁ désigne méthyle et R₄ représente n-hexadécyle (C₁₆) ou n-octadécyle (C₁₈) ou leurs mélanges.

Les polymères non réticulés particulièrement préférés sont obtenus à partir de motifs AMPS de formule (II) et de motifs de formule (III) où x = 8, R₁ désigne le radical méthyle (CH₃) et R₄ = C16-18.

Les polymères pour lesquels X⁺ désigne le sodium ou l'ammonium sont plus particulièrement préférés.

Les polymères amphiphiles préférés conformes à l'invention peuvent être obtenus selon les procédés classiques de polymérisation radicalaire en présence d'un ou plusieurs initiateurs tels que par exemple, l'azobisisobutyronitrile (AIBN), l'azobisdiméthylvaléronitrile, le chlorhydrate de 2,2-azobis-[2-amidinopropane] (ABAH=2,2-AzoBis-[2-Amidinopropane] Hydrochloride), les peroxydes organiques tels que le peroxyde de dilauryle, le peroxyde de benzoyle, l'hydroperoxyde de tert-butyle, etc..., des composés peroxydés minéraux tels que le persulfate de potassium ou d'ammonium, ou H₂O₂ éventuellement en présence de réducteurs.

Les polymères peuvent être notamment obtenus par polymérisation radicalaire en milieu tert-butanol dans lequel ils précipitent. En utilisant la polymérisation par précipitation dans le tert-butanol, il est possible d'obtenir une distribution de la taille des particules du polymère particulièrement favorable pour ses utilisations.

La distribution de la taille des particules du polymère peut être déterminée par exemple par diffraction laser ou analyse d'image. Une distribution intéressante pour ce type de polymère et déterminée par analyse d'image est la suivante : 60,2 % inférieur à 423 microns, 52,0 % inférieur à 212 microns, 26,6 % inférieur à 106 microns, 2,6 % inférieur à 45 microns et 26,6 % supérieur à 850 microns.

La réaction peut être conduite à une température comprise entre 0 et 150°C, de préférence entre 10 et 100°C, soit à pression atmosphérique, soit sous pression réduite. Elle peut aussi être réalisée sous atmosphère inerte, et de préférence sous azote.

Selon ce procédé, on a notamment polymérisé l'acide 2-acrylamido-2-méthylpropane-sulfonique (AMPS) ou l'un de ses sels de sodium ou d'ammonium, avec un ester de l'acide (méth)acrylique et
- d'un alcool en C₁₀-C₁₈ oxyéthyléné par 8 moles d'oxyde d'éthylène (GENAPOL® C-080 de la société HOECHST/CLARIANT),
- d'un alcool oxo en C₁₁ oxyéthyléné par 8 moles d'oxyde d'éthylène (GENAPOL® UD-080 de la société HOECHST/CLARIANT),
- d'un alcool oxo en C₁₁ oxyéthyléné par 7 moles d'oxyde d'éthylène (GENAPOL® UD-070 de la société HOECHST/CLARIANT),
- d'un alcool en C₁₂-C₁₄ oxyéthyléné par 7 moles d'oxyde d'éthylène (GENAPOL® LA-070 de la société HOECHST/CLARIANT),
- d'un alcool en C₁₂-C₁₄ oxyéthyléné par 9 moles d'oxyde d'éthylène (GENAPOL® LA-090 de la société HOECHST/CLARIANT),
- d'un alcool en C₁₂-C₁₄ oxyéthyléné par 11 moles d'oxyde d'éthylène (GENAPOL® LA-110 de la société HOECHST/CLARIANT),
- d'un alcool en C₁₆-C₁₈ oxyéthyléné par 8 moles d'oxyde d'éthylène (GENAPOL® T-080 de la société HOECHST/CLARIANT),
- d'un alcool en C₁₆-C₁₈ oxyéthyléné par 15 moles d'oxyde d'éthylène (GENAPOL^{®} T-150 de la société HOECHST/CLARIANT),
- d'un alcool en C₁₆-C₁₈ oxyéthyléné par 11 moles d'oxyde d'éthylène (GENAPOL^{®} T-110 de la société HOECHST/CLARIANT),
- d'un alcool en C₁₆-C₁₈ oxyéthyléné par 20 moles d'oxyde d'éthylène (GENAPOL^{®} T-200 de la société HOECHST/CLARIANT),
- d'un alcool en C₁₆-C₁₈ oxyéthyléné par 25 moles d'oxyde d'éthylène (GENAPOL^{®} T-250 de la société HOECHST/CLARIANT),
- d'un alcool en C₁₈-C₂₂ oxyéthyléné par 25 moles d'oxyde d'éthylène et/ou d'un alcool iso-C₁₆-C₁₈ oxyéthyléné par 25 moles d'oxyde d'éthylène.

La concentration molaire en % des motifs de formule (II) et des motifs de formule (III) dans les polymères selon l'invention varie en fonction de l'application cosmétique souhaitée et des propriétés rhéologiques de la formulation recherchées. Elle peut varier entre 0,1 et 99,9 % en moles.

Pour les polymères non réticulés les plus hydrophobes, la proportion molaire en motifs de formule (I) ou (III) varie de préférence de 40 à 99,9 %, plus particulièrement de 50 à 90 % et encore plus particulièrement de 60 à 80 %. Pour les polymères non réticulés peu hydrophobes, la proportion molaire en motifs de formule (I) ou (III) varie de préférence de 0,1 à 50 %, plus particulièrement de 1 à 25 % et encore plus particulièrement de 2 à 20 %.

Selon un mode préféré de réalisation de l'invention, le polymère amphiphile utilisé comme polymère non réticulé est un copolymère d'AMPS et de méthacrylate d'alcool en C₁₆-C₁₈ comportant 8 groupes oxyéthylénés, obtenu à partir d'AMPS ou d'un sel d'AMPS et de Genapol T-080 ou de Genapol C-080.

La distribution des monomères dans les polymères de l'invention peut être, par exemple, alternée, bloc (y compris multibloc) ou quelconque.

Selon un autre mode de réalisation de l'invention, les polymères peuvent avoir des chaînes pendantes sensibles à la chaleur et dont la solution aqueuse présente une viscosité qui, au delà d'une certaine température seuil, augmente, ou demeure pratiquement constante quand la température augmente. Il s'agit alors plus particulièrement de polymères dont la solution aqueuse présente une viscosité qui est faible en dessous d'une première température seuil et qui, au dessus de cette première température seuil croît vers un maximum quand la température augmente, et qui, au dessus d'une seconde température seuil décroît à nouveau quand la température augmente. Dans cet optique, on préfère que la viscosité des solutions de polymère en dessous de la première température seuil soit de 5 à 50 %, en particulier de 10 à 30 % de la viscosité maximum à la seconde température seuil. Ces polymères, de préférence, conduisent dans l'eau à un phénomène de démixion par chauffage se traduisant par des courbes présentant, en fonction de la température et de la concentration, un minimum appelé LCST (Lower Critical Solution Temperature). Les viscosités (mesurées à 25°C au viscosimètre Brookfield aiguille 7) des solutions aqueuses à 1 % de tels polymères vont de préférence de 20 000 mPa.s à 100 000 mPa.s et plus particulièrement de 60 000 mPa.s à 70 000 mPa.s.

Les polymères amphiphiles non réticulés conformes à l'invention sont présents dans les compositions dans des quantités allant de 0,01 à 20 % en poids de matière active, plus préférentiellement de 0,1 à 10 % de matière active, encore plus préférentiellement de 0,1 à 5 % en poids de matière active et plus particulièrement encore de 0,5 à 2 % en poids de matière active par rapport au poids total de la composition. De manière plus particulière, les polymères amphiphiles non réticulés utilisés conformément à l'invention sont de manière préférée présents dans la composition de l'invention en une quantité égale ou inférieure à 1 % en poids par rapport au poids total de la composition. Ils peuvent être présents par exemple dans des quantités allant de 0,01 à 1 % en poids de matière active, plus préférentiellement de 0,05 à 0,7 % de matière active, encore plus préférentiellement de 0,1 à 0,6 % en poids de matière active par rapport au poids total de l'émulsion.

De préférence, le polymère amphiphile non réticulé est introduit dans la phase aqueuse de l'émulsion selon l'invention.

La composition selon l'invention comportant au moins 40 % de phase huileuse, la quantité de phase aqueuse va généralement de 20 à 60 % en poids, de préférence de 25 à 55 % en poids et mieux de 30 à 50 % en poids par rapport au poids total de la composition.

La phase aqueuse comprend de l'eau et tout autre composé hydrosoluble éventuellement présent, tels que notamment les solvants et additifs hydrosolubles (par exemple tensioactifs et actifs).

Comme solvants hydrosolubles, on peut citer notamment les alcools inférieurs et les polyols. On entend par alcool inférieur les alcools comportant de 1 à 8 et plus particulièrement de 1 à 6 atomes de carbone, tels que l'éthanol. Comme polyols, on peut citer par exemple la glycérine, le propylène glycol, le butylène glycol et les polyéthylène glycols (PEG-8 par exemple). Ces alcools et/ou polyols peuvent être présents dans la composition en une quantité allant de préférence de 0,1 à 25 % et de 1 à 15 % du poids total de la composition.

La phase huileuse de la composition selon l'invention comprend les huiles et autres corps gras ainsi que les additifs liposolubles (par exemple tensioactifs et actifs). Elle contient au moins une huile démaquillante choisie parmi les huiles hydrocarbonées, les esters gras comportant au moins 8 atomes de carbone, et leurs mélanges.

On entend par « huile hydrocarbonée » toute huile comportant majoritairement des atomes de carbone et d'hydrogène. Comme huiles hydrocarbonées, on peut citer par exemple les huiles de paraffine, volatiles ou non, et leurs dérivés, l'huile de vaseline, les polydécènes, le polyisobutène hydrogéné tel que l'huile de Parléam®, l'isoparaffine hydrogénée, l'isohexadecane, l'isododecane, et leurs mélanges.

On entend par "ester gras" un ester obtenu à partir d'un acide gras et/ou à partir d'un alcool gras. Les esters gras comportent au moins 8 atomes de carbone et de préférence de 9 à 26 atomes de carbone et mieux de 12 à 22 atomes de carbone. L'ester constituant l'huile démaquillante peut être notamment choisi parmi :
(1) les esters obtenus à partir d'acide gras à chaîne droite ou ramifiée comportant au moins 9 atomes de carbone et d'alcools à chaîne droite ou ramifiée comportant de 1 à 17 atomes de carbone ;
(2) les esters obtenus à partir d'acide à chaîne droite ou ramifiée comportant 1 à 17 atomes de carbone et d'alcool gras à chaîne droite ou ramifiée comportant au moins 9 atomes de carbone ;
(3) les esters obtenus à partir de l'acide benzoïque et d'alcool gras à chaîne droite ou ramifiée comportant au moins 9 atomes de carbone ; et
(4) leurs mélanges.

Les esters sont de préférence de mono- ou di-esters.

De manière préférée, l'ester pouvant constituer l'huile démaquillante est choisi dans le groupe des esters qui ne comportent aucune insaturation et/ou aucun groupement éther ou hydroxyle. De façon encore plus avantageuse, il s'agit d'un ester saturé qui ne renferme aucun groupement éther ni hydroxyle.

Ainsi, l'ester pouvant être utilisé comme huile démaquillante de la composition conforme à l'invention peut être notamment choisi dans le groupe comprenant le palmitate d'éthyl-2 hexyle (ou palmitate d'octyle), l'isononanoate d'isononyle, le lactate d'isostéaryle, le myristate d'éthyl-2 hexyle (ou myristate d'octyle), le palmitate d'isopropyle, le myristate d'isopropyle, l'adipate de di-isopropyle, l'adipate de di-octyle, l'hexanoate d'éthyl-2 hexyle, le laurate d'éthyle, le myristate de méthyle, l'octanoate d'octyldodécyle, le néopentanoate d'isodécyle, le myristate d'éthyle, le propionate de myristyle, l'éthyl-2 hexanoate d'éthyl-2 hexyle, l'octanoate d'éthyl-2 hexyle, le caprate/caprylate d'éthyl-2 hexyle, le palmitate de méthyle, le myristate de butyle, le myristate d'isobutyle, le palmitate d'éthyle, le laurate d'isohexyle, le laurate d'hexyle, l'isostéarate d'isopropyle, l'ester d'acide benzoïque et d'alcools en C₁₂-C₁₅ (nom CTFA : C12-15 Alkyl Benzoate), et leurs mélanges.

La quantité d'huile(s) démaquillante(s) peut constituer tout ou une partie de la phase huileuse, et par exemple de 1 à 100 %, de préférence 1 à 90 %, plus préférentiellement de 5 à 80 %, mieux de 5 à 70 % et encore mieux 10 à 70 % du poids total de la phase huileuse.

La phase huileuse peut contenir en outre tous les corps gras et notamment les huiles non démaquillantes autres que celles indiquées ci-dessus, classiquement utilisés dans le domaine cosmétique. Comme autres huiles susceptibles d'être présentes dans la phase huileuse, on peut citer par exemple les huiles d'origine végétale comme l'huile de noyaux d'abricot ; les huiles de synthèse ; les huiles de silicone volatiles ou non volatiles et les huiles fluorées. Les autres corps gras susceptibles d'être présents dans la phase huileuse peuvent être par exemple les acides gras, les alcools gras et les cires.

Comme huiles de silicone, on peut citer par exemple les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ; leurs mélanges.

Selon un mode particulier de réalisation de l'invention, la composition de l'invention comprend au moins une huile démaquillante telle que définie ci-dessus et au moins une huile choisie parmi les huiles de silicone. Plus préférentiellement, la composition comprend au moins un ester et/ou au moins une huile hydrocarbonée et au moins une huile de silicone.

La phase huileuse est présente en une quantité pouvant aller par exemple de 40 à 80 % en poids, de préférence de 45 à 75 % en poids et mieux de 50 à 70 % en poids par rapport au poids total de la composition.

De façon connue, la composition de l'invention peut en outre contenir un ou plusieurs adjuvants, choisis parmi les adjuvants habituels dans le domaine cosmétique, tels que les actifs hydrophiles ou lipophiles, les conservateurs, les antioxydants, les parfums, les solvants, les charges, les fibres, les filtres solaires, les matières colorantes (pigments ou nacres), les ajusteurs de pH (bases ou acides tels que l'acide citrique), les vésicules lipidiques, les polymères autres que ceux indiqués ci-dessus et leurs mélanges. Ces adjuvants sont utilisés dans les proportions habituelles dans le domaine cosmétique ou dermatologique, par exemple de 0,01 à 30 % du poids total de la composition, et ils sont, selon leur nature, introduits dans la phase aqueuse ou dans la phase huileuse de l'émulsion, ou encore dans des vésicules. Ces adjuvants ainsi que leurs concentrations doivent être tels qu'ils ne modifient pas la propriété recherchée pour la composition.

Ainsi, on peut incorporer dans la composition de l'invention, un ou plusieurs polymères, dans des concentrations allant de préférence de 0,05 à 2 % en poids par rapport au poids total de la composition. On peut citer comme exemples de polymères utilisables dans la composition de l'invention :
- les biopolymères polysaccharidiques comme la gomme de xanthane, la gomme de guar, les alginates, les celluloses modifiées ;
- les polymères synthétiques tels que les polyacryliques comme le CARBOPOL 980 commercialisé par la société GOODRICH, les copolymères acrylate/acrylonitrile tels que le HYPAN SS201 commercialisé par la société KINGSTON ;
- les composés inorganiques tels que les smectites, les hectorites modifiées ou non telles que les produits BENTONE commercialisés par la société RHEOX, les produits LAPONITE commercialisés par la société SOUTHERN CLAY PRODUCTS, le produit VEEGUM HS commercialisé par la société R.T. VANDERBILT ;
- et leurs mélanges.

Comme actifs utilisables dans la composition de l'invention, on peut citer en particulier les actifs antiséborrhéïques et antimicrobiens; permettant notamment de traiter les peaux grasses. Cet actif peut notamment être choisi parmi les dérivés de bêta-lactame, les dérivés de quinolone, la ciprofloxacine, la norfloxacine, la tétracycline et ses sels, l'érythromycine et ses sels, l'amikacine et ses sels, le 2,4,4'-trichloro-2'-hydroxy diphényl éther (ou triclosan), le 3,4,4'-trichlorocarbanilide (ou triclocarban), le phénoxyéthanol, le phénoxypropanol, le phénoxyisopropanol, la doxycycline et ses sels, la capréomycine et ses sels, la chlorhexidine et ses sels, la chlortétracycline et ses sels, l'oxytétracycline et ses sels, la clindamycine et ses sels, l'éthambutol et ses sels, l'hexamidine iséthionate, le métronidazole et ses sels, la pentamidine et ses sels, la gentamicine et ses sels, la kanamycine et ses sels, la lincomycine et ses sels, la méthacycline et ses sels, la méthénamine et ses sels, la minocycline et ses sels, la néomycine et ses sels, la netilmicine et ses sels, la paromomycine et ses sels, la streptomycine et ses sels, la tobramycine et ses sels, le miconazole et ses sels, les sels d'amantadine, le para-chloro-méta-xylénol, la nystatine, le tolnaftate, l'acide salicylique et ses sels, l'acide n-octanoyl-5 salicylique et ses sels, le peroxyde de benzoyle, l'acide 3-hydroxy benzoïque, l'acide 4-hydroxy benzoïque, l'acide acétylsalicylique, l'acide 2-hydroxybutanoïque, l'acide 2- hydroxypentanoïque, l'acide 2-hydroxyhexanoïque, l'acide phytique, la N-acétyl-L-cystéine, l'acide lipoïque, l'acide azélaïque, l'acide arachidonique, l'ibuprofène, le naproxène, l'hydrocortisone, l'acétaminophène, le résorcinol, l'octopirox, le chlorhydrate de lidocaïne, le clotrimazole, l'octoxyglycérine, l'octanoylglycine, le caprylylglycol, l'acide 10-hydroxy-2-décanoïque, les sels de zinc tels que le gluconate de zinc, la niacinamide ou vitamine B3 (ou vitamine PP), et leurs mélanges.

Comme actifs utilisables dans la composition de l'invention, on peut citer aussi les agents kératolytiques et anti-vieillissement comme les hydroxyacides (α-hydroxyacides et β-hydroxyacides) tels que l'acide lactique, l'acide glycolique, l'acide citrique, l'acide salicylique et leurs dérivés ; les huiles essentielles ; les vitamines et en particulier le rétinol (vitamine A), l'acide ascorbique (vitamine C), le tocophérol (vitamine E), la niacinamide (vitamine PP ou B3), le panthénol (vitamine B5) et leurs dérivés (esters par exemple) ; les co-enzymes et en particulier le co-enzyme Q10 ou ubiquinone ; les enzymes telles que par exemple les lipases, les protéases, les phospholipases, les cellulases, les peroxydases notamment les lactoperoxydases, les catalases, les superoxyde dismutases et les extraits végétaux contenant les enzymes précitées ; les levures telles que les *Saccharomyces Cerevisiae* ; les stéroïdes ; les anti-oxydants et anti-radicaux libres ; les hydratants tels que les polyols (glycérine, sorbitol, sucres), les hydrolysats de protéine, l'urée et les mélanges en contenant ; les amincissants comme la caféine ; les agents anti-élastase et anticollagénase ; les agents de traitement des peaux grasses ; les extraits végétaux et notamment les extraits de plancton ; les agents tenseurs ; les agents matifiants ; les antitranspirants tels que les sels d'aluminium ; et leurs mélanges.

Comme dérivés d'acide salicylique, on peut citer notamment les acides n-octanoyl-5-salicylique (nom CTFA : Capryloyl Salicylic Acid), n-décanoyl-5-salicylique, n-dodécanoyl-5-salicylique, n-octyl-5-salicylique, n-heptyloxy-5-salicylique, 5-tert-octylsalicylique, 5-butoxy-salicylique, 5-éthoxy-salicylique, 5-méthoxysalicylique, 5-propoxysalicylique, 5-méthylsalicylique, 5-éthylsalicylique, 5-propylsalicylique, et leurs mélanges. Ces acides peuvent être éventuellement salifiés par une base.

Comme exemples de stéroïdes, on peut citer par exemple la déhydroépiandrostérone (ou DHEA), ainsi que (1) ses précurseurs et dérivés biologiques, en particulier les sels et esters de DHEA, tels que le sulfate et le salicylate de DHEA, la 7-hydroxy DHEA, la 7-céto DHEA, les esters de 7-hydroxy et 7-céto DHEA, notamment la 3-beta-acétoxy-7-oxo DHEA, et (2) ses précurseurs et dérivés chimiques, en particulier les sapogénines telles que la diosgénine ou l'hécogénine, et/ou leurs dérivés tels que l'acétate d'hécogénine, et/ou les extraits naturels en contenant et notamment les extraits de Dioscorées, tels que l'igname sauvage (Wild Yam).

Selon un mode particulier de réalisation de l'invention, l'actif est choisi parmi les hydroxy-acides, les vitamines, les extraits de plancton et leurs mélanges.

Les fibres utilisables dans la composition de l'invention peuvent être des fibres hydrophiles ou hydrophobes, d'origine synthétique ou naturelle, minérale ou organique. Les fibres peuvent être par exemple celles utilisées dans la fabrication des textiles et notamment des fibres de soie, de coton, de laine, de lin, de cellulose extraites notamment du bois, des légumes ou des algues, de polyamide (Nylon®), de cellulose modifiée (rayonne, viscose, acétate notamment d'acétate de rayonne), de poly-p-phénylène téréphtalamide notamment de Kevlar®, d'acrylique notamment de polyméthacrylate de méthyle ou de poly 2-hydroxyéthyl méthacrylate, de polyoléfine et notamment de polyéthylène ou de polypropylène, de verre, de silice, d'aramide, de carbone notamment sous forme graphite, de Téflon®, de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères tels que ceux mentionnés ci-dessus, comme des fibres de polyamide/polyester, et leurs mélanges. On peut aussi utiliser les fibres synthétiques résorbables utilisées en chirurgie, comme les fibres préparées à partir d'acide glycolique et de caprolactone (Monocryl de la société Johnson & Johnson) ; les fibres synthétiques résorbables du type copolymère d'acide lactique et d'acide glycolique (Vicryl de la société Johnson & Johnson) ; les fibres de polyester téréphtalique (Ethibond de la société Johnson & Johnson), et les fils d'acier inoxydable (Acier de la société Johnson & Johnson). On peut aussi utiliser un mélange de ces différentes fibres. Ces fibres peuvent être présentes dans des quantités allant de 0 à 20% en poids et de préférence de 0,5 à 10 % en poids par rapport au poids total de la composition.

La composition de l'invention peut contenir en outre des charges en vue de modifier la texture de la composition. Comme charges qui peuvent être utilisées dans la composition de l'invention, on peut citer par exemple, outre les pigments, la poudre de silice ; le talc ; les particules de polyamide et notamment celles vendues sous la dénomination ORGASOL par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les poudres de matériaux organiques naturels tels que les amidons de maïs, de blé ou de riz, réticulés ou non, telles que les poudres d'amidon réticulé par l'anhydride octénylsuccinique, commercialisées sous la dénomination DRY-FLO par la société National Starch ; les microbilles de résine de silicone telles que celles commercialisées sous la dénomination TOSPEARL par la société Toshiba Silicone ; et leurs mélanges. Ces charges peuvent être présentes dans des quantités allant de 0 à 40% en poids et de préférence de 1 à 10 % en poids par rapport au poids total de la composition.

Les filtres solaires (ou filtres U.V.) éventuellement présents dans la composition peuvent être choisis parmi les filtres organiques, les filtres physiques tels que les oxydes de titane ou de zinc et leurs mélanges.

Les filtres UV organiques plus particulièrement préférés sont choisis parmi les composés suivants :
- Les dérivés salicyliques et notamment l'éthylhexyl salicylate (ou salicylate d'éthyl hexyle) vendu sous le nom commercial NEO HELIOPAN OS par HAARMANN et REIMER ;
- Les dérivés du dibenzoylméthane et notamment le Butyl méthoxydibenzoylmethane vendu notamment sous le nom commercial PARSOL 1789 par HOFFMANN LA ROCHE ;
- Les dérivés cinnamiques et notamment l'éthylhexyl méthoxycinnamate vendu notamment sous le nom commercial PARSOL MCX par HOFFMANN LA ROCHE;
- Les dérivés de β,β-diphénylacrylate et notamment l'octocrylène (α-cyano-β,β-diphénylacrylate de 2-éthylhexyle) vendu notamment sous le nom commercial UVINUL N539 par BASF ;
- L'acide phenylbenzimidazole sulfonique ;
- Les dérivés du benzylidène camphre, et notamment l'acide téréphthalylidène dicamphosulfonique (Terephthalylidene Dicamphor Sulfonic acid) fabriqué sous le nom MEXORYL SX par CHIMEX, et le 4-méthylbenzylidène camphre vendu sous le nom commercial EUSOLEX 6300 par MERCK ;
- Les dérivés de la benzophénone, et notamment la Benzophenone-3 ou Oxybenzone, vendue sous le nom commercial UVINUL M40 par BASF ; la Benzophenone-4 vendue sous le nom commercial UVINUL MS40 par BASF ; la Benzophenone-5 ;
- Les dérivés du phényl benzimidazole, et notamment le Benzimidazilate vendu sous le nom commercial NEO HELIOPAN AP par HAARMANN et REIMER ;
- Les dérivés de la triazine, et notamment l'anisotriazine vendu sous le nom commercial TINOSORB S par CIBA GEIGY ; l'éthylhexyl triazone vendu notamment sous le nom commercial UVINUL T150 par BASF ; et le diéthylhexyl Butamido Triazone vendu sous le nom commercial UVASORB HEB par SIGMA 3V ;
- Le méthylène bis-benzotriazolyl tétraméthylbutylphenol ;
- Les dérivés du phényl benzotriazole, et notamment le Drometrizole Trisiloxane vendu sous le nom commercial SILATRIZOLE par RHODIA CHIMIE ;
- et leurs mélanges.

Comme filtres physiques pouvant être ajoutés dans la composition de l'invention, on peut citer par exemple les pigments et nano-pigments d'oxydes métalliques, enrobés ou non enrobés, notamment les oxydes de titane, de fer, de zirconium, de zinc ou de cérium, et leurs mélanges, ces oxydes pouvant être sous forme de micro- ou nanoparticules (nanopigments), éventuellement enrobées.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas ou substantiellement pas, altérées par l'addition envisagée.

Les compositions selon l'invention peuvent être plus ou moins fluides et elles peuvent donc se présenter sous forme de lotion, de lait ou sous forme de crème plus ou moins épaisse. Elles peuvent constituer notamment des produits de démaquillage et/ou de nettoyage de la peau, des lèvres et/ou des yeux.

La présente invention a encore pour objet un procédé cosmétique de démaquillage et/ou de nettoyage de la peau, des lèvres et/ou des yeux, caractérisé par le fait que l'on applique sur la peau, les lèvres et/ou les yeux, une composition telle que définie ci-dessus.

L'invention a aussi pour objet l'utilisation cosmétique de la composition telle que définie ci-dessus pour le démaquillage et/ou le nettoyage de la peau, des lèvres et/ou des yeux.

L'exemple ci-après de compositions selon l'invention est donné à titre d'illustration et sans caractère limitatif. Les quantités y sont données en % en poids, sauf mention contraire.

### Exemples 1 à 4 selon l'invention

Le procédé de préparation des compositions des exemples 1 à 4 est le suivant : on disperse A1 dans l'eau avec A4 sous agitation, puis on ajoute les autres constituants de la phase A ; on émulsionne B dans A à 25°C sous agitation mécanique jusqu'à former une émulsion blanche, lisse et brillante.

| Exemples Emulsion H/E | | n°1 | n°2 | n°3 | n°4 |
|---|---|---|---|---|---|
| *Phase A aqueuse* | - A1 - copolymère AMPS / méthacrylate d'alcool C16-18 (8 OE) | 0.4 | 0.4 | 0.4 | 0.6 |
| | - A2 - eau | qsp 100 | qsp 100 | qsp 100 | qsp 100 |
| | - A3 - glycérine | 3 | 3 | 3 | 3 |
| | - A4 - base (soude) | 0.07 | 0.07 | 0.07 | 0.0105 |
| | - A5 - conservateur | 0.1 | 0.1 | 0.1 | 0.1 |
| *Phase B huileuse* | - Cyclopentasiloxane | 12 | 36 | - | - |
| | - Huile de vaseline | 31.8 | | | |
| | - Palmitate | 16.2 | | 24 | 24 |
| | d'isopropyle | | | | |
| | -Isohexadécane | - | 24 | | |
| | - Isododécane | - | | 36 | 36 |
| *Contrôles à 24 heures* | | pH = 7,23 | pH = 6,91 | pH = 7,43 | pH = 7 |
| | | viscosité = 19 poises (=1,9 Pa.s) | viscosité = 464 cPoises (= 0,464 Pa.s) | viscosité = 15 poises (= 1,5 Pa.s) | viscosité = 4,6 poises (= 0,46 Pa.s) |
| | | taille des globules huileux = 7µm | taille des globules huileux = 20pm | taille des globules huileux = 28µm | |
| | | Emulsion fine, homogène, blanche et brillante | Emulsion plus fluide, homogène, blanche | Emulsion fine et homogène. | Emulsion d'aspect blanc, homogène |
| *Stabilité après 2 mois de recul à 4°C, 25°C et 45°C* | | Stable | Stable | Stable | Stable |

Ces émulsions présentent les avantages suivants :
➢ Elles sont donc stables après un recul de 2 mois à différentes températures (4°C, 25°C, 45°C).
➢ Elles peuvent avoir une viscosité assez élevée (environ 15 poises, soit 1,5 Pa.s au Rhéomat 180 à 25°C - 200 s⁻¹). Dans ce cas-là, la composition n'est pas un lait mais une crème souple, et elle présente l'avantage de ne pas couler à l'application sur la peau (exemples n°5 et 7).
➢ Plusieurs natures d'huiles sont compatibles, aussi bien les silicones que les alcanes ou les esters ou d'autres
➢ Ces émulsions sont stables sans ajout d'autres agents de surface (tensioactif émulsionnant, co-tensioactif tels des alcools gras)

### Pouvoir démaquillant :

On a testé le pouvoir démaquillant des exemples 5 à 8 indiqués ci-dessus, en utilisant un fond de teint sans transfert pour vérifier l'efficacité du démaquillage.

Le test est réalisé de la manière suivante :
5) On applique le fond de teint sans transfert "Air Wear" de L'Oréal Paris, en une quantité de 50 mg sur une surface définie de 4x4 cm² de l'avant-bras.
6) En parallèle, les formules démaquillantes sont pesées (100 mg) et déposées sur un coton.
7) Après séchage du maquillage (environ 15 minutes), on applique par 5 mouvements (du haut vers le bas) le coton imbibé de démaquillant sur la zone définie maquillée.
8) Ensuite, on compare le démaquillage obtenu par rapport à la peau nue.

Dans ce test, les formules n°1, 2, 3 ont montré un excellent démaquillage, la formule n°4 un démaquillage correcte.

L'efficacité du démaquillant, le confort et l'évaluation sensorielle de la formule n°3 ont été aussi testés sur un panel de 27 femmes qui l'ont utilisée durant 10 jours. Il est ressorti de ce test que le produit avait un pouvoir démaquillant jugé bon, des aspects cosmétiques très bons, un rinçage facile, et un bon confort.

## Revendications

1. Composition cosmétique de nettoyage et/ou de démaquillage, comprenant une phase huileuse dispersée dans une phase aqueuse, **caractérisée par le fait qu'**elle comprend au moins un polymère amphiphile comportant au pleins un monomère à insaturation éthylénique à groupement sulfonique, sous forme libre ou partiellement ou totalement neutralisée, non réticulé, comportant au moins deux parties latérales hydrophobes, et que la phase huileuse comprend au moins une huile démaquillante et constitue au moins 40 % en poids par rapport au poids total de la composition, le polymère amphiphile étant choisi parmi les copolymères amphiphiles d'AMPS et d'au moins un monomère hydrophobe à insaturation éthylénique comportant au moins une partie hydrophobe ayant de 6 à 50 atomes de carbone et de préférence 6 à 30 atomes de carbone.

2. Composition selon la revendication précédente, **caractérisée par le fait que** la partie hydrophobe du polymère amphiphile comporte de 6 à.22 atomes de carbone.

3. Composition selon l'une quelconque des revendications précédentes, **caractérisée par le fait que** le polymère amphiphile est neutralisé partiellement ou totalement par une base minérale ou organique.

4. Composition selon l'une quelconque des revendications précédentes, **caractérisée par le fait que** le polymère amphiphile a un poids moléculaire moyen en nombre allant de 1000 à 20 000 000 g/mole, de préférence de 20 000 à 5 000 000 g/mole.

5. Composition selon l'une quelconque des revendications précédentes, **caractérisée par le fait qu'**une solution aqueuse à 1 % en poids dudit polymère présente à la température de 25°C une viscosité mesurée au viscosimètre Brookfield, aiguille 7, allant de 20 000 mPa.s à 100 000 mPa.s

6. Composition selon l'une quelconque des revendications précédente, **caractérisée par le fait que** le polymère amphiphile est préparé par polymérisation radicalaire par précipitation dans le tert-butanol.

7. Composition selon l'une des revendications précédentes, **caractérisée par le fait que** le monomère hydrophobe à insaturation éthylénique est choisi parmi les acrylates, alkylacrylates, acrylamides ou alkylacrylamides de formule (I) suivante : 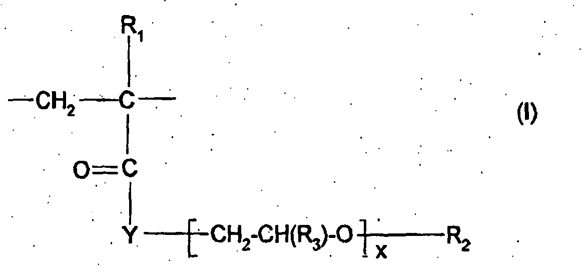 dans laquelle R₁ et R₃, identiques ou différents, désignent un atome d'hydrogène ou un radical alkyle linéaire ou ramifié en C₁-C₆ ; Y désigne O ou NH ; R₂ désigne un radical hydrocarboné hydrophobe comportant de 6 à 50 atomes de carbone ; x désigne un nombre de moles d'oxyde d'alkylène et varie de 0 à 100.

8. Composition selon la revendication précédente, **caractérisée par le fait que** le radical hydrophobe R₂ est choisi parmi les radicaux alkyles en C₆-C₁₈, linéaires, ramifiés ou cycliques ; les radicaux alkylperfluorés en C₆-C₁₈ ; le radical cholestéryle ou un reste d'ester de cholestérol ; les groupes polycycliques aromatiques.

9. Composition selon la revendication 7 ou 8 **caractérisée par le fait que** le monomère de formule (I) comporte au moins un motif oxyde d'alkylène (x ≥ 1).

10. Composition selon la revendication précédente, **caractérisée par le fait que** le nombre de motifs oxyde d'alkylène varie de 3 à 100.

11. Composition selon la revendication précédente, **caractérisée par le fait que** le nombre de motifs oxyde d'alkylène varie de 3 à 50.

12. Composition selon l'une quelconque des revendications 7 à 11, **caractérisée par le fait que** le monomère de formule (I) comporte uniquement de motifs oxyde d'éthylène.

13. Composition selon l'une des revendications précédentes, **caractérisée par le fait que** le polymère amphiphile d'AMPS est choisi parmi :
- les copolymères, réticulés ou non réticulés, neutralisés ou non, comportant de 15 à 60 % en poids de motifs AMPS et de 40 à 85 % en poids de motifs (C₈-C₁₆)alkyl(méth)acrylamide ou de motifs (C₈-C₁₆)alkyl(meth)acrylate, par rapport au polymère ;
- les terpolymères comportant de 10 à 90 % en mole de motifs acrylamide, de 0,1 à 10% en mole de motifs AMPS et de 5 à 80 % en mole de motifs N-(C₆-C₁₈)alkylacrylamide, par rapport au polymère ;
- les copolymères, réticulés ou non réticulés, d'AMPS partiellement ou totalement neutralisé et de méthacrylate de n-dodécyle, de n-hexadécyle et/ou n-octadécyle ;
- les copolymères, réticulés ou non réticules, d'AMPS partiellement ou totalement neutralisé et de n-dodécylméthacrylamide.

14. Composition selon l'une des revendications précédentes, **caractérisée par le fait que** le polymère amphiphile d'AMPS est choisi parmi les copolymères constitués de motifs acide 2-acrylamido-2-méthylpropane-sulfonique (AMPS) de formule (II) suivante : 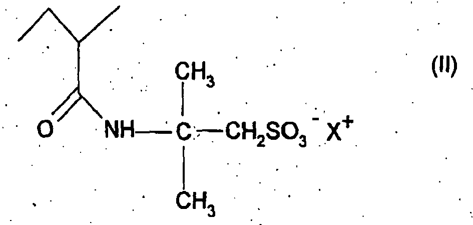 dans laquelle X⁺ est un proton, un cation de métal alcalin, un cation alcalino-terreux ou l'ion ammonium,
et de motifs de formule (III) suivante : 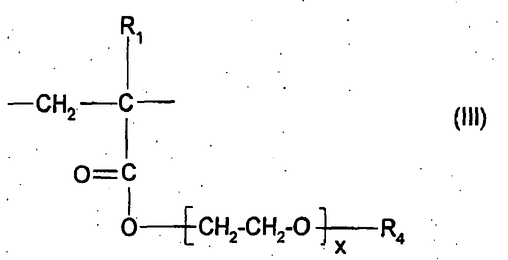 dans laquelle x désigne un nombre entier variant de 3 à 100 ; R₁ a la même signification que celle indiquée ci-dessus dans la formule (I) et R₄ désigne un alkyle linéaire ou ramifié en C₅-C₂₂.

15. Composition selon la revendication précédente, **caractérisée par le fait que** x = 25, R₁ est méthyle et R₄ est n-dodécyle, n-hexadécyle, n-octadécyte.

16. Composition selon la revendication 14, **caractérisée par le fait que** x = 8, R₁ désigne le radical méthyle et R₄ est C16-18.

17. Composition selon l'une quelconque des revendications précédentes, **caractérisée par le fait que** la quantité de polymère(s) amphiphile(s) va de 0,01 à 20 % en poids de matière active et de préférence de 0,1 à 10 % en poids de matière active, par rapport au poids total de la composition.

18. Composition selon l'une quelconque des revendications précédentes, **caractérisée par le fait que** la quantité de phase huileuse va de 40 à 80 % en poids et de préférence de 45 à 75 % en poids par rapport au poids total de la composition.

19. Composition selon l'une quelconque des revendications précédentes, **caractérisée par le fait que** l'huile démaquillante est choisie parmi les huiles hydrocarbonées, les esters d'acide gras comportant au moins 8 atomes de carbone, et leurs mélanges.

20. Composition selon l'une quelconque des revendications précédentes, **caractérisée par le fait que** l'huile démaquillante est choisie parmi les huiles de paraffine, volatiles ou non, et leurs dérivés, l'huile de vaseline, les polydécènes, le polyisobutène hydrogéné tel que l'huile de parléam, l'isoparaffine hydrogénée, l'isohexadecane, l'isododecane, les esters obtenus à partir d'acide gras à chaîne droite ou ramifiée comportant au moins 9 atomes de carbone et d'alcools à chaîne droite ou ramifiée comportant de 1 à 17 atomes de carbone ; les esters obtenus à partir d'acide gras à chaîne droite ou ramifiée comportant 1 à 17 atomes de carbone et d'alcool gras à chaîne droite ou ramifiée comportant au moins 9 atomes de carbone ; les esters obtenus à partir de l'acide benzoïque et d'alcool gras à chaîne droite ou ramifiée comportant au moins 9 atomes de carbone ; et leurs mélanges.

21. Composition selon l'une quelconque des revendications précédentes, **caractérisée par le fait que** la phase huileuse comprend en outre au moins une huile de silicone.

22. Composition selon l'une quelconque des revendications précédentes, **caractérisée par le fait que** la quantité d'huile(s) démaquillante(s) représente de 1 à 100 % et mieux de 10 à 80 % du poids total de la phase huileuse.

23. Composition selon l'une quelconque des revendications précédentes, **caractérisée par le fait qu'**elle contient en outre un ou plusieurs adjuvants choisis parmi les actifs hydrophiles ou lipophiles, les conservateurs, les antioxydants, les parfums, les solvants, les charges, les fibres, les filtres solaires, les matières colorantes, les polymères, les agents basiques ou acides, les vésicules lipidiques, et leurs mélanges.

24. Utilisation cosmétique de la composition selon l'une quelconque des revendications précédents pour le démaquillage et/ou le nettoyage de la peau, des lèvres et/ou des yeux.

25. Procédé cosmétique de démaquillage et/ou de nettoyage de la peau, des lèvres et/ou des yeux, **caractérisé en ce que** l'on applique sur la peau, les lèvres et/ou les yeux; une composition selon l'une quelconque des revendications 1 à 23.

## Claims

1. Cosmetic cleansing and/or makeup-removing composition, comprising an oily phase dispersed in an aqueous phase, **characterized in that** it comprises at least one noncrosslinked amphiphilic polymer comprising at least one monomer comprising ethylenic unsaturation comprising a sulphonic group, in the free or partially or completely neutralized form, comprising at least two hydrophobic side parts, and **in that** the oily phase comprises at least one make-up-removing oil and constitutes at least 40% by weight with respect to the total weight of the composition, said amphiphilic polymer being chosen from amphiphilic copolymers of AMPS and of at least one ethylenically unsaturated hydrophobic monomer comprising at least one hydrophobic portion containing from 6 to 50 carbon atoms and preferably from 6 to 30 carbon atoms.

2. Composition according to the preceding claim, **characterized in that** the hydrophobic portion of the amphiphilic polymer contains from 6 to 22 carbon atoms.

3. Composition according to any one of the preceding claims, **characterized in that** the amphiphilic polymer is partially or totally neutralized with a mineral or organic base.

4. Composition according to any one of the preceding claims, **characterized in that** the amphiphilic polymer has a number-average molecular weight ranging from 1000 to 20 000 000 g/mol and preferably from 20 000 to 5 000 000 g/mol.

5. Composition according to any one of the preceding claims, **characterized in that** an aqueous 1% by weight solution of the said polymer has, at a temperature of 25°C, a viscosity, measured using a Brookfield viscometer with a No. 7 needle, ranging from 20 000 mPa.s to 100 000 mPa.s.

6. Composition according to any one of the preceding claims, **characterized in that** the amphiphilic polymer is prepared by free-radical precipitation polymerization in tert-butanol.

7. Composition according to the any one of the preceding claims, **characterized in that** the ethylenically unsaturated hydrophobic monomer is chosen from the acrylates, alkylacrylates, acrylamides or alkylacrylamides of formula (I) below: 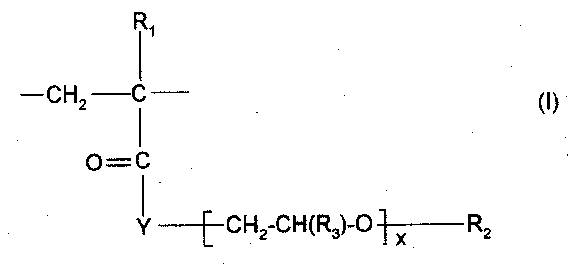 in which R₁ and R₃, which may be identical or different, denote a hydrogen atom or a linear or branched C₁-C₆ alkyl radical; Y denotes O or NH; R₂ denotes a hydrophobic hydrocarbon-based radical containing from 6 to 50 carbon atoms; x denotes a number of moles of alkylene oxide and ranges from 0 to 100.

8. Composition according to the preceding claim, **characterized in that** the hydrophobic radical R₂ is chosen from linear, branched or cyclic C₆-C₁₈ alkyl radicals; C₆-C₁₈ alkylperfluoro radicals; the cholesteryl radical or a cholesterol ester residue; aromatic polycyclic groups.

9. Composition according to Claim 7 or 8, **characterized in that** the monomer of formula (I) comprises at least one alkylene oxide unit (x ≥ 1).

10. Composition according to the preceding claim, **characterized in that** the number of alkylene oxide units ranges from 3 to 100.

11. Composition according to the preceding claim, **characterized in that** the number of alkylene oxide units ranges from 3 to 50.

12. Composition according to any one of Claims 7 to 11, **characterized in that** the monomer of formula (I) comprises only ethylene oxide units.

13. Composition according to any one of the preceding claims, **characterized in that** the amphiphilic AMPS polymer is chosen from:
- crosslinked or non-crosslinked, neutralized or non-neutralized copolymers comprising from 15% to 60% by weight of AMPS units and from 40% to 85% by weight of (C₈-C₁₆)alkyl (meth)acrylamide units or of (C₈-C₁₆)alkyl (meth)acrylate units, relative to the polymer;
- terpolymers comprising from 10 mol% to 90 mol% of acrylamide units, from 0.1 mol% to 10 mol% of AMPS units and from 5 mol% to 80 mol% of N-(C₆-C₁₈)alkyl-acrylamide units, relative to the polymer;
- crosslinked or non-crosslinked copolymers of partially or totally neutralized AMPS and of n-dodecyl, n-hexadecyl and/or n-octadecyl methacrylate,
- crosslinked or non-crosslinked copolymers of partially or totally neutralized AMPS and of N-dodecyl-methacrylamide.

14. Composition according to any one of the preceding claims, **characterized in that** the amphiphilic AMPS polymer is chosen from copolymers consisting of 2-acrylamido-2-methylpropanesulfonic acid (AMPS) units of formula (II) below: 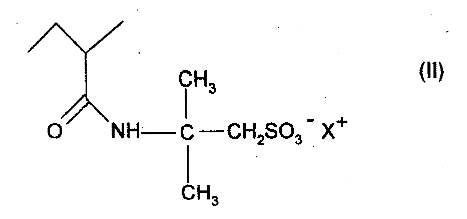 in which X⁺ is a proton, an alkali metal cation, an alkaline-earth metal cation or the ammonium ion,
and of units of formula (III) below: 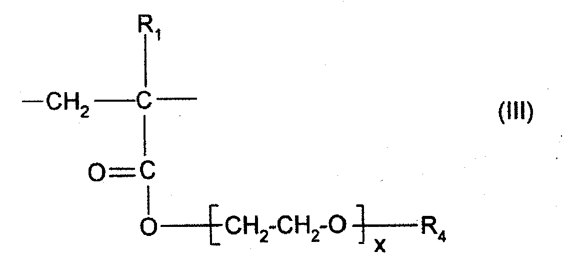 in which x denotes an integer ranging from 3 to 100; R₁ has the same meaning as that given above in formula (I) and R₄ denotes a linear or branched C₆-C₂₂ alkyl.

15. Composition according to the preceding claim, **characterized in that** x = 25, R₁ is methyl and R₄ is n-dodecyl, n-hexadecyl or n-octadecyl.

16. Composition according to Claim 14, **characterized in that** x = 8, R₁ denotes a methyl radical and R₄ is C16-18.

17. Composition according to any one of the preceding claims, **characterized in that** the amount of amphiphilic polymer(s) ranges from 0.01% to 20% by weight of active material and preferably from 0.1% to 10% by weight of active material relative to the total weight of the composition.

18. Composition according to any one of the preceding claims, **characterized in that** the amount of oily phase ranges from 40% to 80% by weight and preferably from 45% to 75% by weight relative to the total weight of the composition.

19. Composition according to any one of the preceding claims, **characterized in that** the makeup-removing oil is chosen from hydrocarbon-based oils and fatty acid esters containing at least 8 carbon atoms, and mixtures thereof.

20. Composition according to any one of the preceding claims, **characterized in that** the makeup-removing oil is chosen from volatile or non-volatile liquid paraffins, and derivatives thereof, liquid petroleum jelly, polydecenes, hydrogenated polyisobutene such as Parleam oil, hydrogenated isoparaffin, isohexadecane, isododecane, the esters obtained from straight- or branched-chain fatty acids containing at least 9 carbon atoms and from straight- or branched-chain alcohols containing from 1 to 17 carbon atoms; the esters obtained from straight- or branched-chain fatty acids containing 1 to 17 carbon atoms and from straight- or branched-chain fatty alcohols containing at least 9 carbon atoms; the esters obtained from benzoic acid and from straight- or branched-chain fatty alcohols containing at least 9 carbon atoms; and mixtures thereof.

21. Composition according to any one of the preceding claims, **characterized in that** the oily phase also comprises at least one silicone oil.

22. Composition according to any one of the preceding claims, **characterized in that** the amount of makeup-removing oil(s) represents from 1% to 100% and better still from 10% to 80% of the total weight of the oily phase.

23. Composition according to any one of the preceding claims, **characterized in that** it also contains one or more adjuvants chosen from hydrophilic or lipophilic active agents, preserving agents, antioxidants, fragrances, solvents, fillers, fibres, sunscreens, dyestuffs, polymers, acidic or basic agents, and lipid vesicles, and mixtures thereof.

24. Cosmetic use of the composition according to any one of the preceding claims for removing makeup from and/or for cleansing the skin, the lips and/or the eyes.

25. Cosmetic process for removing makeup from and/or for cleansing the skin, the lips and/or the eyes, **characterized in that** a composition according to any one of Claims 1 to 23 is applied to the skin, the lips and/or the eyes.

## Patentansprüche

1. Kosmetische Zusammensetzung für die Reinigung und/oder zum Abschminken, die eine in einer wässrigen Phase dispergierte Ölphase enthält, **dadurch gekennzeichnet, dass** sie mindestens ein nicht vernetztes amphiphiles Polymer enthält, das mindestens ein Monomer mit ethylenisch ungesättigter Bindung und einer in freier Form oder ganz oder teilweise neutralisiert vorliegenden Sulfonsäuregruppe enthält und mindestens zwei hydrophobe Seitenbereiche aufweist, und **dadurch**, dass die Ölphase mindestens ein Öl zum Abschminken enthält und mindestens 40 Gew.-%, bezogen auf das Gesamtgewicht der Zusammensetzung, ausmacht, wobei das amphiphile Polymer unter den amphiphilen Copolymeren von AMPS und mindestens einem hydrophoben Monomer mit ethylenisch ungesättigter Bindung ausgewählt ist, das mindestens einen hydrophoben Bereich mit 6 bis 50 Kohlenstoffatomen und vorzugsweise 6 bis 30 Kohlenstoffatomen aufweist.

2. Zusammensetzung nach dem vorhergehenden Anspruch, **dadurch gekennzeichnet, dass** der hydrophobe Bereich des amphiphilen Polymers 6 bis 22 Kohlenstoffatome enthält.

3. Zusammensetzung nach einem der vorhergehenden Ansprüche, **dadurch gekennzeichnet, dass** das amphiphile Polymer ganz oder teilweise mit einer anorganischen Base oder einer organischen Base neutralisiert ist.

4. Zusammensetzung nach einem der vorhergehenden Ansprüche, **dadurch gekennzeichnet, dass** das amphiphile Polymer eine zahlenmittlere Molmasse von 1.000 bis 20.000.000 g/mol und vorzugsweise 20.000 bis 5.000.000 g/mol aufweist.

5. Zusammensetzung nach einem der vorhergehenden Ansprüche, **dadurch gekennzeichnet, dass** eine wässrige Lösung von 1 Gew.-% des Polymers eine bei einer Temperatur von 25 °C mit einem Brookfield-Viskosimeter, Nadel 7, gemessene Viskosität von 20.000 bis 100.000 mPa·s aufweist.

6. Zusammensetzung nach einem der vorhergehenden Ansprüche, **dadurch gekennzeichnet, dass** das amphiphile Polymer durch radikalische Polymerisation mit Fällung in *tert*-Butanol hergestellt wird.

7. Zusammensetzung nach einem der vorhergehenden Ansprüche, **dadurch gekennzeichnet, dass** das hydrophobe Monomer mit ethylenisch ungesättigter Bindung unter den Acrylaten, Alkylacrylaten, Acrylamiden oder Alkylacrylamiden der folgenden Formel (I) ausgewählt ist: 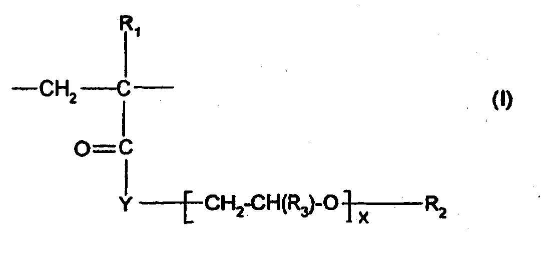 worin die Gruppen R₁ und R₃, die gleich oder verschieden sind, ein Wasserstoffatom oder eine geradkettige oder verzweigte C₁₋₆-Alkylgruppe bedeuten; Y O oder NH bedeutet; R₂ eine hydrophobe Kohlenwasserstoffgruppe mit 6 bis 50 Kohlenstoffatomen ist; x die Anzahl der Mol Alkylenoxid angibt und im Bereich von 0 bis 100 liegt.

8. Zusammensetzung nach dem vorhergehenden Anspruch, **dadurch gekennzeichnet, dass** die hydrophobe Gruppe R₂ unter den geradkettigen, verzweigten oder cyclischen C₆₋₁₈-Alkylgruppen; C₆₋₁₈-Perfluoralkylgruppen; der Cholesterylgruppe oder einem Cholesterinesterrest; und aromatischen polycyclischen Gruppen ausgewählt ist.

9. Zusammensetzung nach Anspruch 7 oder 8, **dadurch gekennzeichnet, dass** das Monomer der Formel (I) mindestens eine Alkylenoxideinheit aufweist (x ≥ 1).

10. Zusammensetzung nach dem vorhergehenden Anspruch, **dadurch gekennzeichnet, dass** die Anzahl der Alkylenoxideinheiten im Bereich von 3 bis 100 liegt.

11. Zusammensetzung nach dem vorhergehenden Anspruch, **dadurch gekennzeichnet, dass** die Anzahl der Alkylenoxidgruppen im Bereich von 3 bis 50 liegt.

12. Zusammensetzung nach einem der Ansprüche 7 bis 11, **dadurch gekennzeichnet, dass** das Monomer der Formel (I) ausschließlich Ethylenoxidgruppen aufweist.

13. Zusammensetzung nach einem der vorhergehenden Ansprüche, **dadurch gekennzeichnet, dass** das amphiphile AMPS-Polymer ausgewählt ist unter:
- vernetzten oder nicht vernetzten, neutralisierten oder nicht neutralisierten Copolymeren, die 15 bis 60 Gew.-% AMPS-Einheiten und 40 bis 85 Gew.-% (C₈₋₁₆)Alkyl(meth)acrylamideinheiten oder (C₈₋₁₆)Alkyl(meth)acrylateinheiten, bezogen auf das Polymer, enthalten;
- Terpolymeren, die 10 bis 90 Mol-% Acrylamideinheiten, 0,1 bis 10 Mol-% AMPS-Einheiten und 5 bis 80 Mol-% N-(C₆₋₁₈)-Alkylacrylamideinheiten, bezogen auf das Polymer, enthalten;
- vernetzten oder nicht vernetzten Copolymeren von ganz oder teilweise neutralisierter AMPS und n-Dodecylmethacrylat, n-Hexadecylmethacrylat und/oder n-Octadecylmethacylat;
- vernetzten oder nicht vernetzten Copolymeren von ganz oder teilweise neutralisierter AMPS und n-Dodecylmethacrylamid.

14. Zusammensetzung nach einem der vorhergehenden Ansprüche, **dadurch gekennzeichnet, dass** das amphiphile AMPS-Polymer unter den Copolymeren ausgewählt ist, die aus 2-Acrylamido-2-methylpropan-sulfonsäureeinheiten (AMPS) der folgenden Formel (II) : 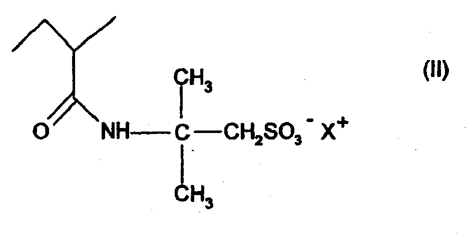 worin X⁺ ein Proton, ein Alkalimetallkation, ein Erdalkalimetallkation oder das Ammoniumion ist, und Einheiten der folgenden Formel (III) bestehen: 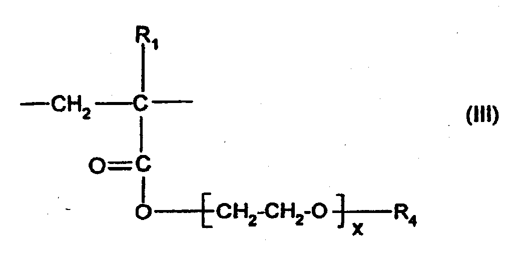 worin x eine ganze Zahl von 3 bis 100 bedeutet; R₁ die oben in Formel (I) angegebenen Bedeutungen hat und R₄ eine geradkettige oder verzweigte C₆₋₂₂-Alkylkette ist.

15. Zusammensetzung nach dem vorhergehenden Anspruch, **dadurch gekennzeichnet, dass** x = 25, R₁ bedeutet Methyl und R₄ ist n-Dodecyl, n-Hexadecyl, n-Octadecyl.

16. Zusammensetzung nach Anspruch 14, **dadurch gekennzeichnet, dass** x = 8, R₁ bedeutet Methyl und R₄ weist 16 bis 18 Kohlenstoffatome auf.

17. Zusammensetzung nach einem der vorhergehenden Ansprüche, **dadurch gekennzeichnet, dass** der Mengenanteil des amphiphilen Polymers oder der amphiphilen Polymere im Bereich von 0,01 bis 20 Gew.-% wirksame Substanz und vorzugsweise 0,1 bis 10 Gew.-% wirksame Substanz, bezogen auf das Gesamtgewicht der Zusammensetzung, liegt.

18. Zusammensetzung nach einem der vorhergehenden Ansprüche, **dadurch gekennzeichnet, dass** der Mengenanteil der Ölphase im Bereich von 40 bis 80 Gew.-% und vorzugsweise 45 bis 75 Gew.-%, bezogen auf das Gesamtgewicht der Zusammensetzung, liegt.

19. Zusammensetzung nach einem der vorhergehenden Ansprüche, **dadurch gekennzeichnet, dass** das Öl zum Abschminken unter den Kohlenwasserstoffölen, Fettsäureestern mit mindestens 8 Kohlenstoffatomen und deren Gemischen ausgewählt ist.

20. Zusammensetzung nach einem der vorhergehenden Ansprüche, **dadurch gekennzeichnet, dass** das Öl zum Abschminken unter den flüchtigen oder nichtflüchtige Paraffinölen und deren Derivaten, Vaselineöl, Polydecenen, hydriertem Polyisobuten, wie Parleamöl, hydriertem Isoparaffin, Isohexadecan, Isododecan, Estern, die aus einer Fettsäure mit gerader oder verzweigter Kette und mindestens 9 Kohlenstoffatomen und Alkoholen mit gerader oder verzweigter Kette mit 1 bis 17 Kohlenstoffatomen gebildet werden; Estern, die aus einer Fettsäure mit gerader oder verzweigter Kette mit 1 bis 17 Kohlenstoffatomen und einem Fettalkohol mit gerader oder verzweigter Kette und mindestens 9 Kohlenstoffatomen gebildet werden; Estern, die aus Benzoesäure und einem Fettalkohol mit gerader oder verzweigter Kette mit mindestens 9 Kohlenstoffatomen gebildet werden; und deren Gemischen ausgewählt ist.

21. Zusammensetzung nach einem der vorhergehenden Ansprüche, **dadurch gekennzeichnet, dass** die Ölphase ferner mindestens ein Siliconöl enthält.

22. Zusammensetzung nach einem der vorhergehenden Ansprüche, **dadurch gekennzeichnet, dass** der Mengenanteil des Öls oder der Öle zum Abschminken 1 bis 100 % und besser 10 bis 80 % des Gesamtgewichts der Ölphase ausmacht.

23. Zusammensetzung nach einem der vorhergehenden Ansprüche, **dadurch gekennzeichnet, dass** sie ferner einen oder mehrere Zusatzstoffe enthält, die unter den hydrophilen oder lipophilen Wirkstoffen, Konservierungsmitteln, Antioxidantien, Parfums, Lösungsmitteln, Füllstoffen, Fasern, Sonnenschutzfiltern, Farbmitteln, Polymeren, basischen Stoffen oder Säuren, Lipidvesikeln und deren Gemischen ausgewählt sind.

24. Kosmetische Verwendung der Zusammensetzung nach einem der vorhergehen Ansprüche zum Abschminken und/oder Reinigen der Haut, der Lippen und/oder der Augen.

25. Kosmetisches Verfahren zum Abschminken und/oder Reinigen der Haut, der Lippen und/oder der Augen, das **dadurch gekennzeichnet ist, dass** auf die Haut, die Lippen und/oder die Augen eine Zusammensetzung nach einem der Ansprüche 1 bis 23 aufgebracht wird.
